# EUROPÄISCHE PATENTSCHRIFT

(11) **EP 0 668 048 B1**
(45) Veröffentlichungstag und Bekanntmachung des Hinweises auf die Patenterteilung: **12.04.2000**
(21) Anmeldenummer: 95100740.0
(22) Anmeldetag: 20.01.1995
(51) Int. Cl.: A61B 5/15

(54) **Blutentnahmevorrichtung**
Blood sampling device
Dispositif de prise de sang

(30) Priorität: 29.01.1994 DE 4402690
(43) Veröffentlichungstag der Anmeldung: 23.08.1995
(73) Patentinhaber: Sarstedt AG & Co., 51588 Nümbrecht (DE)
(72) Erfinder: Sarstedt, Walter, D-51588 Nümbrecht-Rommelsdorf (DE)
(74) Vertreter: Pollmeier, Felix, Dipl.-Ing.

(56) Entgegenhaltungen:
- EP-A- 0 026 679
- EP-A- 0 342 653
- FR-A- 1 462 681
- US-A- 3 135 261

## Beschreibung

Die Erfindung betrifft eine Blutentnahmevorrichtung, die ein Entnahmeröhrchen aufweist, in dessen vorderes Röhrchenende ein durchstechbarer Stopfen einer auf das Entnahmeröhrchen aufgesetzten und dieses verschließenden Stopfenkappe eingreift, der die Stopfenkappe in dem Entnahmeröhrchen hält, wobei die Stopfenkappe das Entnahmeröhrchen mit einem Kragen übergreift, und die eine mit einer Führungshülse die Stopfenkappe aufnehmende Doppelkanüle aufweist.

Bei einer aus der DE 30 49 503 C bekannten Blutentnahmevorrichtung besitzt die das Entnahmeröhrchen an seinem vorderen Ende abschließende Kappe einen zylindrischen, in axialer Richtung vorstehenden Ansatz. Der Ansatz ist an seinem vorderen Ende durch einen durchstechbaren Verschlußstopfen abgeschlossen, der auf einer mit einer Mittelbohrung versehenen Stirnplatte des Ansatzes aufliegt und von einem am vorderen Ende umgebördelten Kragen gehalten wird. Die rohrförmige Führungshülse, die an ihrem vorderen Ende in einem Halter eine doppelendige, beiderseits mit einer Schneidkante versehene Kanüle trägt, deren aus der Führungshülse vorstehendes Ende zur Einführung in eine Vene dient, während ihr hinteres Ende so weit in die Führungshülse hineinragt, daß es beim Ansetzen der Führungshülse an das Entnahmeröhrchen den Verschlußstopfen durchsticht, ist axial verschiebbar und drehbar auf dem Ansatz angeordnet. Das hintere, in die Führungshülse hineinragende Kanülenende wird von einem sackartigen Schlauch (Ventilgummi) solcher Länge eingehüllt, daß die Schneidkante des hinteren Kanülenendes bei gestrecktem Schlauch noch nicht dessen Boden berührt.

Zur Verbindung der Doppelkanüle mit dem Ansatz ist letzterer mit einem seitlich vorstehenden Haltenocken versehen, dem Axialschlitze in der Führungshülse zugeordnet sind. Mittels des in einen der über den Umfang verteilten Axialschlitze eingeführten Haltenocken läßt sich eine bajonettverschlußartige Drehverriegelung der in lockerer Passung auf den Ansatz sitzenden Führungshülse der Doppelkanüle erreichen. Eine derartige Drehverriegelung erhöht allerdings den Herstellungsaufwand und verteuert damit entsprechend die Blutentnahmevorrichtung.

Ein durch die EP 0 129 029 B bekanntgewordenes Entnahmeröhrchen einer Blutentnahmevorrichtung ist mit einer flexiblen Stopfenkappe der eingangs genannten Art verschlossen, d.h. sie ist mit einem Stopfen in das offene Ende des Röhrchens eingesteckt und weist einen das Röhrchen - vorzugsweise mit Spiel - übergreifenden Kragen auf. Die das Entnahmeröhrchen kragenartig ummantelnde Stopfenkappe verhindert, daß beim Abziehen des gefüllten Entnahmeröhrchens Blut verspritzen kann. Um einen Aerosoleffekt zu verhindern, ist es notwendig, daß der Stopfen zusammen mit der Stopfenkappe abgezogen wird. Das bedeutet, daß die Stopfenkappe aus einem eine Verformung der Anlagefläche verhindernden Material bestehen muß. Die Flexibilität der Stopfenkappe wird eingeschränkt durch die Haltefunktion mit dem Stopfen. Die im praktischen Einsatz zu diesem Entnahmeröhrchen gehörende Führungshülse ist von erheblich größerem Durchmesser als die Stopfenkappe, und von einer das Blut bei einem Patienten abnehmenden Person wird eine gewisse Geschicklichkeit vorausgesetzt, um trotz des großen Führungsspiels eine ordnungsgemäße, patientenschonende Blutentnahme durchführen zu können. Da aber keine besonderen Rast- bzw. Halteverbindungen vorgesehen sind, läßt sich die bekannte Blutentnahmevorrichtung mit einem entsprechend verringerten Aufwand herstellen.

Schließlich ist aus der DE 29 48 653 C eine Blutentnahmevorrichtung mit ebenfalls einer einen in axialer Richtung vorstehenden Ansatz aufweisenden Verschlußkappe bekannt. Die Führungshülse der Doppelkanüle ist auf ein Anpassungsstück des Ansatzes der auf ein Außengewinde des Blutentnahmeröhrchens aufgeschraubten Verschlußkappe aufschiebbar. Sowohl die Verschlußkappe mit dem Ansatz und dem Anpassungsstück als auch die Führungshülse bestehen aus thermoplastischem Kunststoff. Das Anpassungsstück besitzt hinter dem Verschlußstopfen einen Rohrstutzen mit einer konischen Bohrung, die zur Halterung der Doppelkanüle auf den mit der Verschlußkappe einstückigen, entsprechend konischen Ansatz aufgesetzt ist. Bei dieser Art der Verbindung hat sich gezeigt, daß sie entweder zu stramm oder unerwünscht locker ist; ein zu strammer Sitz kann aufgrund des damit verbundenen ruckartigen Überganges dazu führen, daß beim Aufsetzen des Entnahmeröhrchens die Kanüle durch die Vene des Patienten hindurchgedrückt wird. Ein weiterer Nachteil dieser Art der Entnahmevorrichtung stellt sich dann ein, wenn die Führungshülse verformbar ausgebildet ist. Wird dann nämlich nach dem Einbringen der Kanüle in die Vene des Patienten das Blutentnahmeröhrchen mit der Führungshülse gekoppelt, muß neben dem Durchdringungswiderstand von Nadel und Stopfen, die bei der die Kanüle aufweisenden Fixierungshülse, z.B. durch Daumen und Zeigefinger, aufgebracht wird, auch die radiale Kraft der unrunden Führungshülse überwunden werden. Da sich eine gleichmäßige Kraft nicht aufbringen läßt, ist ein ruckfreies Ankoppeln nicht möglich.

Der Erfindung liegt die Aufgabe zugrunde, eine Blutentnahmevorrichtung der eingangs genannten Art zu schaffen, die ohne Rast- oder Schraubverbindungen einen einerseits sicheren Halt von Führungshülse und Stopfenkappe ermöglicht und dabei andererseits auf einfache, schonende Weise erlaubt, bei in der Vene verbleibender Kanüle mehrere Entnahmeröhrchen ruckfrei mit dieser zu koppeln bzw. zu verbinden und Blut in mehrere Entnahmeröhrchen abzufüllen.

Diese Aufgabe wird erfindungsgemäß dadurch gelöst, daß die Stopfenkappe oder die Führungshülse mit mindestens einer, die Stopfenkappe beim Einschieben in die Führungshülse im Kragenbereich zumindest mittelbar verformenden Erhöhung ausgebildet ist und die Führungshülse steif und zumindest die Erhöhung der Stopfenkappe nachgiebig, vorzugsweise weichelastisch ausgebildet ist. Somit läßt sich auf verblüffend einfache Weise eine selbstklemmende Anordnung der Führungshülse direkt auf dem Kragen der Stopfenkappe erreichen. Es wurde erkannt und entsprechend ausgenutzt, daß sich im Zusammenwirken der steifen, vorzugsweise aus duroplastischem Kunststoff bestehenden und ggf. an ihrem Außenmantel durch Versteifungsrippen noch zusätzlich verstärkten Führungshülse und der weichelastischen Stopfenkappe und/oder nachgiebigen Erhöhung beim Ineinanderfügen bzw. -schieben von Führungshülse und Stopfenkappe aufgrund der mindestens einen Erhöhung, und zwar gleichgültig, ob diese am Innenmantel der steifem Führungshülse oder am Außenmantel des Kragens der Stopfenkappe angeordnet ist, ein die selbstklemmende Verbindung bzw. Halterung bewirkender Kräftefluß erreichen läßt. Es kann nämlich die Verformung der weichelastischen Stopfenkappe und/oder der Erhöhung zur starren Verbindung von Doppelkanüle und Entnahmeröhrchen ausgenutzt werden, wobei sich sogar größere Toleranzen überbrücken lassen.

Dieser Effekt läßt sich begünstigen, wenn zwischen dem Kragen der Stopfenkappe und dem Entnahmeröhrchen ein Spiel vorhanden ist, das außerdem unterstützt, den Aerosoleffekt zu verhindern. Gleichwohl ist es möglich, die Stopfenkappe so zu gestalten, daß der Kragen an dem Blutentnahmeröhrchen anliegt. Um eine die Halterung bewirkende Verformung zu ermöglichen, müßte dann auf jeden Fall die Erhöhung - bzw. die Erhöhungen - weichelastisch bzw. nachgiebig gestaltet sein, beispielsweise durch eine entsprechende Materialauswahl und/oder durch in der Erhöhung vorgesehene Hohlräume bzw. Ausnehmungen, die die Nachgiebigkeit ermöglichen bzw. unterstützen. Die Erhöhung läßt sich z.B. in Form einer komischen Verdickung bzw. umlaufenden Wulst, einer punktförmigem Materialanhäufung oder einer in ihrem Endabschnitt in eine Keilfläche übergehenden Längsrippe ausbilden. Sind mehrere Erhöhungen vorgesehen, so sollten diese über den Umfang der Stopfenkappe bzw. des Kragens verteilt angeordnet werden.

Dabei ist eine solche Gestaltung der Erhöhung bzw. Erhöhungen möglich, daß sie beim Ineinanderschieben von Führungshülse und Stopfenkappe erst ab einem bestimmten Umfang im Anlage mit der Führungshülse bzw. der Stopfenkappe gelangen, nämlich dann, wenn das hintere Ende der Doppelkanüle gerade in das Ventilgummi ein- bzw. dieses durchdringt. Sobald die Durchdringungskraft wegfällt, wird die weichelastische Stopfenkappe und/oder die Erhöhung verformt und damit die starre, sebstklemmende Halterung erreicht. Die Erhöhung kann hierbei so ausgelegt werden, daß sich beim Ineinanderschieben die Kraft bzw. der Gegendruck langsam aufbaut, d.h. allmählich gesteigert wird und beim Herausziehen, z.B. wenn mehrere Proben genommen werden müssen, entsprechend allmählich und langsam abbaut. Es treten keine ruckartigen Übergänge auf, so daß verhindert wird, daß die Kanüle die Vene des Patienten durchdringt.

Weitere Merkmale und Vorteile der Erfindung ergeben sich aus dem Patentanspruch und der nachfolgenden Beschreibung, in der in den Zeichnungen schematisch dargestellte Ausführungsbeispiele des Gegenstandes der Erfindung näher erläutert sind. Es zeigen:
- Fig. 1: als Einzelheit die Ansicht auf das vordere, durch eine Stopfenkappe verschlossene Ende eines Entnahmeröhrchens einer Blutentnahmevorrichtung;
- Fig. 2: den Gegenstand nach Fig. 1 im Querschnitt;
- Fig. 3: im Querschnitt eine Blutentnahmevorrichtung, bei der das in Fig. 1 gezeigte Entnahmeröhrchen über die Stopfenkappe selbstklemmend in einer Führungshülse einer auf die Stopfenkappe aufgesteckten Doppelkanüle angeordnet ist;
- Fig. 4: im Querschnitt das vordere Ende eines durch eine andere Ausführung einer Stopfenkappe verschlossenen Entnahmeröhrchens;
- Fig. 5: im Querschnitt das vordere Ende eines durch eine weitere Ausführung einer Stopfenkappe verschlossenen Entnahmeröhrchens; und
- Fig. 6: als Einzelheit der Fig. 5 in vergrößerter Darstellung den hinteren, mit einer Erhöhung ausgebildeten Bereich der Stopfenkappe, in der Zeichnungsebene nach unten weggeklappt gezeigt.

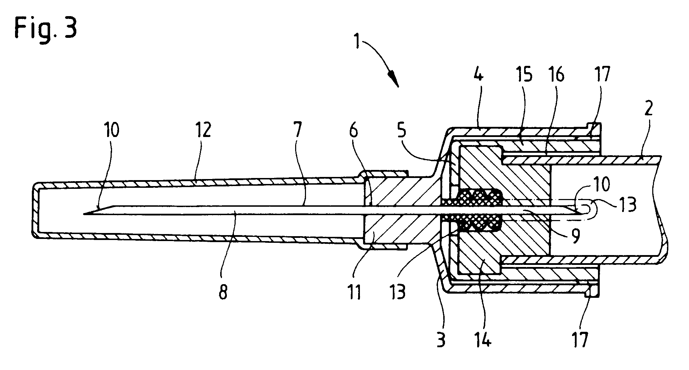

Eine Blutentnahmevorrichtung 1 umfaßt gemäß Fig. 3 ein Entnahmeröhrchen 2 und eine Doppelkanüle 3, die mit einer Führungshülse 4 auf eine das vordere Ende des Entnahmeröhrchens 2 verschließende Stopfenkappe 5 aufgesteckt ist. Die Führungshülse 4 besteht aus einem steifen, duroplastischen Kunststoffmaterial. Die Doppelkanüle 3 ist mit einem Nadel- bzw. Kanülenhalter 6 bestückt, der die Kanüle 7 aufnimmt, deren vorderes und hinteres Kanülenende 8 bzw. 9 angeschärft, d.h. mit Schneidkanten 10 versehen ist. Das vordere Kanülenende 8 dient zur Einführung in eine Vene; solange die Doppelkanüle 3 bzw. die Blutentnahmevorrichtung 1 nicht in Gebrauch ist, wird es von einer auf einem zylindrischen Ansatz 11 der Führungshülse 4 angeordneten Hülse 12 geschützt. Das hintere Kanülendende 9 ragt über den Kanülenhalter 6 hinaus in die Führungshülse 4 hinein; es wird von einem schlauchartigen Ventilgummi 13 solcher Länge umschlossen, daß die Schneidkante 10 des hinteren Kanülenendes 9 bei gestreckten Ventilgummi 13 noch nicht dessen Boden berührt. Das Entnahmeröhrchen 2 kann solcher Art sein, daß es entweder nach dem Saugkolben- oder dem Vakuumprinzip arbeitet.

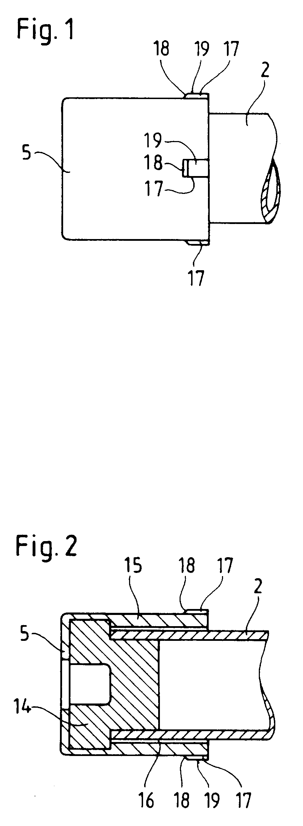

Die aus einem weichelastischen Kunststoffmaterial bestehende Stopfenkappe 5 ist über einen in das Entnahmeröhrchen 2 eingreifenden Stopfen 14 aus Gummi in dem Röhrchen gehalten. Sie besitzt - wie in Figur 2 gezeigt ist - einen Kragen 15, der mit einem geringen Spiel 16 die Außenwand des Entnahmeröhrchens 2 übergreift. An der Außenseite bzw. dem -mantel der Stopfenkappe 5 sind über den Umfang verteilt mehrere Erhöhungen 17 angeordnet, die rippenartig, in Form von Keilschrägen 18, die dann in einen geraden Abschnitt 19 übergehen, ausgebildet sind.

Zur Blutentnahme werden die Doppelkanüle 3 und das Entnahmeröhrchen 2 ineinandergeschoben, d.h. die steife Führungshülse 4 auf die weichelastische Stopfenkappe 5 aufgeschoben bzw. -gesteckt, wie in Figur 3 dargestellt. Dabei trifft zunächst der Boden des Ventilgummis 13 auf den Gummistopfen 14 auf, wobei sich beim weiteren Einfügen das Ventilgummi 13 zieharmonikaartig - wie in Figur 3 angedeutet - zusammenschiebt und die Schneidkante 10 des hinteren Kanülenendes 9 in Kontakt mit dem Boden des Ventigummis 13 gelangt. Das weitere Ineinanderschieben bewirkt, daß die Schneidkante 10 des hinteren Kanülenendes 9 zunächst das Ventilgummi 13 und danach den Gummistopfen 14 durchsticht.

Die den Umfang der weichelastischen Stopfenkappe 5 ab einem bestimmten Bereich vergrößernden Erhöhungen 17 sind so angeordnet und ausgelegt, daß in diesem Moment, d.h. sobald die Durchdringungskraft wegfällt, die Erhöhungen 17 über die ansteigenden Keilschrägen 18 allmählich und zunehmend mehr zur Anlage an die Führungshülse gelangen, worauf sich eine - von den Spiel 16 zwischen der Stopfenkappe 5 und dem Entnahmeröhrchen 2 unterstützte-Verformung der Stopfenkappe 5 einstellt. Diese bewirkt einen sebstklemmenden Verbund von Doppelkanüle 3 und Entnahmeröhrchen 2, und zwar über die auf die Stopfenkappe 5 aufgesteckte Führungshülse 4. Die Keilschrägen 18 begünstigen dabei, daß sich beim Ineinanderschieben ein langsamer Kraftaufbau mit sich entsprechend allmählich steigerndem Gegendruck und beim Herausziehen ein entsprechend schonender Kraftabbau einstellt, wodurch sich ruckartige Übergänge vermeiden lassen. Das erleichtert es, in einer für den Patienten schonenden Weise mehrere Blutproben mit derselben Doppelkanüle zu entnehmen, deren vorderes Kanülenende 8 während des Wechselns des Entnahmeröhrchens 2 in der Vene des Patienten verbleibt.

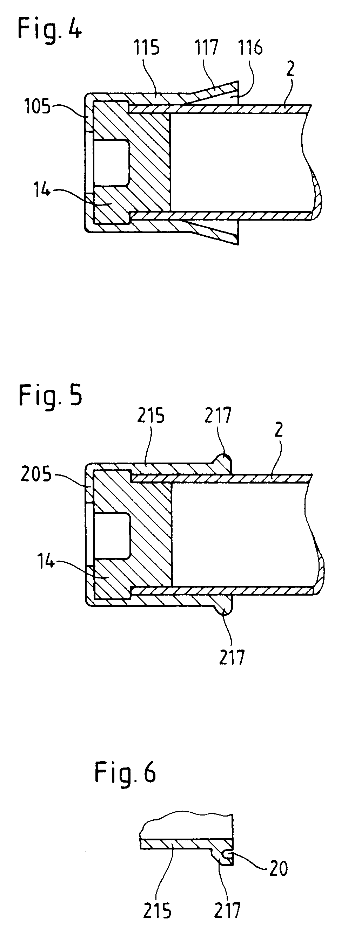

Eine in Figur 4 gezeigte, sich lediglich hinsichtlich der Gestaltung der Erhöhung 117 von der Ausführung nach den Figuren 1 bis 3 unterscheidende Stopfenkappe 105 weist einen Kragen 115 auf, der dem oberen Ende des Entnahmeröhrchens 2 zunächst ohne Spiel anliegt, bevor er dann in einen sich zum Ende hin weitenden, die Erhöhung 117 bildenden, das Entnahmeröhrchen mit einem zunehmend größeren Spiel 116 übergreifenden Endkonus übergeht. Sobald beim Ineinanderschieben von Führungshülse 4 (vgl. Fig. 3) und Entnahmeröhrchen 2 die - an ihrer Innenwandung gegebenenfalls mit Erhöhungen versehene - Führungshülse 4 in den Bereich der konischen Erhöhung 117 gelangt, stellt sich auch in diesem Fall eine Verformung der weichelastischen Stopfenkappe 105 ein, nämlich im Bereich der konischen Erhöhung 117. Weiterhin wird der schon zuvor beschriebene langsame Kraftaufbau beim Ineinanderschieben und entsprechend schonende Kraftabbau beim Herausziehen erreicht, d.h. ohne ruckartige Übergänge zu verursachen.

Eine weitere, ebenfalls auf dem zuvor beschriebenen Prinzip zum ruckfreien Ineinanderschieben und Herausziehen basierende Ausführung einer Stopfenkappe 205 ist in Figur 5 dargestellt. Der Kragen 215 dieser Stopfenkappe liegt dem Außenmantel des Entnahmeröhrchens 2 ohne Spiel an. Die die Verformung und damit die selbstklemmende Halterung der Führungshülse 4 der Doppelkanüle 3 auf der Stopfenkappe 205 gewährleistende Weichelastizität ist hier in den Bereich der bei dieser Ausgestaltung als Nocken oder umlaufende Wulst aus weichem Material ausgebildeten Erhöhung 217 verlegt. Um die Weichelastizität bzw. Nachgiebigkeit der Erhöhung 217 (Nocken, umlaufende Wulst,etc.) zu unterstützen, kann sie - wie in Figur 6 schematisch gezeigt - durch einen Hohlraum 20 noch zusätzlich weich gemacht werden. Die selbstklemmende Halterung der Führungshülse 4 auf der Stopfenkappe 205 wird somit erreicht, sobald die aufgesteckte Führungshülse 4 sich über die Erhöhung 217 schiebt.

## Patentansprüche

1. Blutentnahmevorrichtung, die ein Eintnahmeröhrchen (2) aufweist, in dessen vorderes Röhrchenende ein durchstechbarer Stopfen (14) einer auf das Entnahmeröhrchen (2) aufgesetzten und dieses verschließenden Stopfenkappe (5) eingreift, der die Stopfenkappe (5) in dem Entnahmeröhrchen (2) hält, wobei die Stopfenkappe (5) das Entnahmeröhrchen (2) mit einem Kragen (15) übergreift, und die eine mit einer Führungshülse (4) die Stopfenkappe (5) aufnehmende Doppelkanüle (3) aufweist,
**dadurch gekennzeichnet**,
daß die Stopfenkappe (5, 105, 205) oder die Führungshülse (4) mindestens eine, die Stopfenkappe (5, 105, 205) beim Einschieben in die Führungshülse (4) im Kragenbereich zumindest mittelbar verformende Erhöhung (17, 117, 217) aufweist und die Führungshülse (4) steif und zumindest die Erhöhung (17, 117, 217) der Stopfenkappe (5, 105, 205) oder der Führungshülse (4) nachgiebig, vorzugsweise weichelastisch, ausgebildet ist.

## Claims

1. Blood withdrawal device, which comprises a withdrawal tubelet (2) in the front tubelet end of which engages a stopper (14), which can be plugged through, of a stopper cap (5) placed on and closing the withdrawal tubelet (2), which the stopper cap (5) holds in the withdrawal tubelet (2), wherein the stopper cap (5) engages over the withdrawal tubelet (2) by a collar (15), and which comprises a double cannula (3) receiving the stopper cap (15) by a guide sleeve (4), characterised in that the stopper cap (5, 105, 205) or the guide sleeve (4) has at least one elevation (17, 117, 217) deforming the stopper cap (15, 105, 205) at least indirectly on pushing into the guide sleeve (4), and the guide sleeve (4) is constructed to be rigid and at the least the elevation (17, 117, 217) of the stopper cap (5, 105, 205) or the guide sleeve (4) to be yielding, preferably soft-elastic.

## Revendications

1. Dispositif de prise de sang comprenant un tube de prélèvement (2) dans l'extrémité antérieure de laquelle s'engage un bouchon (14) susceptible d'être transpercé, d'un capuchon (5) de bouchon, coiffé sur le tube de prélèvement (2) et fermant celui-ci, ledit bouchon retenant le capuchon (5) dans le tube de prélèvement (2), le capuchon (5) chevauchant le tube de prélèvement (2) avec une collerette (16), et comprenant une canule double (3) recevant avec une douille de guidage (4) le capuchon (5), caractérisé en ce que le capuchon (5, 105, 205) de bouchon ou la douille de guidage (4) présentant au moins relief (17, 117, 217) déformant au moins indirectement le capuchon (5, 105, 205) dans la région de la collerette lors de l'enfoncement dans la douille de guidage (4), et en ce qu'au moins le relief (17, 117, 217) du capuchon (5, 105, 205) de bouchon ou de la douille de guidage (4) est réalisé flexible, de préférence souple.
